# EUROPEAN PATENT SPECIFICATION

(11) **EP 0 586 555 B1**
(45) Date of publication and mention of the grant of the patent: **08.04.1998**
(21) Application number: 92912904.7
(22) Date of filing: 29.05.1992
(51) Int. Cl.: A61B 17/02, A61B 1/32

(54) **ENDOSCOPIC INFLATABLE RETRACTION DEVICES**
AUFBLASBARE ZURÜCKHALTEVORRICHTUNGEN FÜR ENDOSKOPISCHE EINGRIFFE
DISPOSITIFS ENDOSCOPIQUES ET GONFLABLES DE RETRACTION

(30) Priority: 29.05.1991 US 706781; 19.11.1991 US 794590
(43) Date of publication of application: 16.03.1994
(60) Divisional application: 97202079.6
(73) Proprietor: ORIGIN MEDSYSTEMS, INC., Menlo Park, CA 94025 (US)
(72) Inventor: MOLL, Frederic, H., San Francisco, CA 94118 (US); CHIN, Albert, K., Palo Alto, CA 94303 (US); GADACZ, Thomas, R., Augusta, GA 30909 (US)
(74) Representative: Schmitz, Jean-Marie
(86) International application number: PCT/US92/04507
(87) International publication number: WO 92/21295

(56) References cited:
- EP-A- 0 246 086
- DE-A- 2 847 633
- FR-A- 2 668 695
- US-A- 3 831 587
- US-A- 3 863 639
- US-A- 4 291 687
- US-A- 4 775 371

## Description

The present invention concerns an apparatus for use inside the body for retracting a first tissue from a second tissue according to the precharacterizing portion of claim 1.

The invention relates to devices for use in laparoscopic surgery, in particular, to devices that provide retraction of an organ to gain access to treat or observe a tissue.

Laparoscopy dates back to the turn of the 20th Century. Early laparoscopic techniques were used primarily for diagnostic purposes to view the internal organs, without the necessity of conventional surgery. Since the 1930s, laparoscopy has been used for sterilization and, more recently, for the suturing of hernias. US-A-4,919,152 and 4,944,443 are concerned with techniques for suturing hernias. Another very recent innovation is the use of laparoscopic surgery for removing the gallbladder.

Published International Application No. WO-A 92/21294 under the Patent Cooperation Treaty, describes an apparatus and method wherein the abdominal wall is lifted away from the underlying abdominal organs by an inflatable device which is introduced laparoscopically and, once in place, inflated to engage and lift an extensive area of the abdominal wall.

Even when such lifting techniques are used, it is still necessary to retract other organs to gain access to the organ or tissue to be treated or observed. In other procedures, to gain access to the organ or tissue to be treated or observed, it is necessary to separate the organ to be treated from tissue surrounding it. For example, to be able to observe the outer surface of the heart, the outer surface of the heart has to be separated from the pericardium.

Published International Application No. WO-A 92/21293 under the Patent Cooperation Treaty, describes inflatable retraction devices that retract organs or tissues by means of an inflatable chamber. The retraction device is introduced in a collapsed state into the body through a small incision, and, once in place, inflated to engage an extensive area of the organ or tissue to be retracted, and to gently retract or displace the organ or tissue without damaging it. During laparoscopic treatment and observation procedures, the retraction device retains its expanded condition, and hence its ability to provide retraction, while providing access for surgical instruments through itself to the organ or tissue being treated or observed, or allowing an organ or tissue to be brought inside itself for observation or treatment.

The publication DE-A-2 847 633 describes an apparatus for use inside a body comprising an inflatable chamber means for applying a force between a first organ and a second organ when in an inflated state, the inflatable chamber means being insertable in collapsed state, and an inflating means for inflating the inflatable chamber means when the latter is in place within the body.

The inflatable retraction devices described in WO-A 92/21293 are placed in the body through a small incision, and, when inflated, retract the organ by pushing against other adjacent organs and tissues. The force exterted by the retraction device against other organs or tissues within the body cavity can sometimes cause trauma and even damage to the other organs. Hence, it is sometimes preferably to provide a retraction device that can introduced into the body through a small incision, and which provides retraction without exerting a force against other adjacent organs and tissues.

To provide a retraction from outside to body, current laparoscopic procedures use several small metal or plastic retractors inserted though a plurality of incisions. The retractors are fixed to a suitable bar to hold them in place once the desired amount of retraction has been achieved. Because such retractors have a relatively small surface area (the retractors have to be small enough to fit through a small incision), they tend to damage and/or cause trauma to the retracted organs. Moreover, the required plurality of incisions in the body wall undoes some of the advantage of using laparoscopic techniques.

The apparatus for use inside the body for retracting a first tissue from a second tissue is defined in the characterizing portion of claim 1.

The present invention relates to inflatable retraction devices that mechanically retract organs and tissues to provide access to treat or observe other organs or tissues. In the following description, the word "organ" will be used to mean an organ or a tissue that is retracted by the retraction device. The word "treat" will be used to mean both treat and observe, and the word "treatment" will be used to mean both treatment and observation. The word "tissue" or the phrase "tissue to be treated" will both he used to mean the organ or the tissue that is treated after the organ has been retracted.

An inflatable retraction device according to a first aspect of the invention is used in the body to retract a first organ from a second organ to gain access to a tissue adjacent to the first organ. The inflatable retraction device comprises an inflatable chamber for applying a force between the first organ and the second to retract the first organ from the second organ. The inflatable chamber is adapted for insertion in a collapsed state into the body through a laparoscopic incision, and has a thin, flexible envelope. The apparatus also includes a device for selectively inflating the inflatable chamber to an expanded state while the inflatable chamber is in place within the body.

The inflatable retraction device is used in applications in which it can be placed so that it retracts the organ without obstructing access to the tissue being treated. Treatment of the tissue is carried out working around the retraction device. Surgical instruments are passed into the body through a second small incision, and pass around the outside of the retraction device to reach the tissue.

In a method of using an inflatable retraction device according to the present invention to retract a first organ from a second organ in the body in the course of treating a tissue adjacent to the first organ, an inflatable retraction device is having a collapsed state and an expanded state is provided. A small incision is made in the body and the inflatable retraction device in the collapsed state is introduced into the body through the small incision. The inflatable retraction device in the collapsed state is placed between the first organ and the second organ, and is inflated into the expanded state to apply a force between the first organ and the second organ to retract the first organ from the second organ.

In order that the invention may be fully understood, reference is made on the accompanying drawings, wherein
Figure 1A shows a free balloon inflatable retraction device according to the invention in its expanded state.
Figure 1B shows a free balloon inflatable retraction device according to the invention in its collapsed state.
Figure 2A is a longitudinal cross section of the abdomen showing a free balloon inflatable retraction device according to the invention in its collapsed state inserted into the abdomen through a small incision.
Figure 2B is a longitudinal cross-section of the abdomen showing a free balloon inflatable retraction device according to the invention in its expanded state providing access for an endoscope to the gall bladder.
Figure 2C is a partially cut-away plan view of the abdomen showing a free balloon inflatable retraction device according to the invention in its expanded state providing access for an endoscope to the gall bladder.

### (a) Free Balloon Inflatable Retraction Device

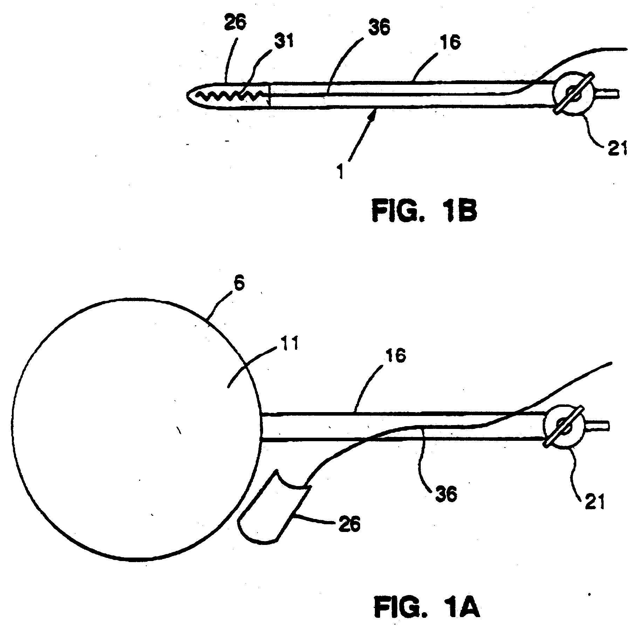

Figures 1A and 1B show a free balloon inflatable retraction device according to the present invention. The free balloon inflatable retraction device is inserted in a collapsed state into a part of the body, such as the abdomen, adjacent to the organ to be retracted. The free balloon inflatable retraction device is inserted into the body through a trocar inserted into a single small incision about 10 - 20 mm long in the body wall. Once in place, the free balloon retractor is inflated into an expanded state to retract the organ.

Figure 1A shows the inflatable retraction device 1 in its expanded state. The envelope 6 encloses the inflatable chamber 11. The envelope 6 is made of a relatively inelastic and tough film of a plastic such as Mylar®, polyethylene, or polyurethane. The preferred material for the envelope is a polyethylene and nylon composite. The thickness of the envelope is typically from 13 to 130 µm (0.5 to 5 mils). The envelope can be a polyhedral structure constructed from two segmented, substantially flat pieces of plastic film, which gives the inflatable chamber a substantially polyhedral shape. Alternatively, two non-segmented, substantially flat pieces of plastic film can be used to make a relatively flat inflatable chamber. In a further alternative, two curved pieces of plastic film can be used to give the inflatable chamber a substantially spherical or spheroidal shape. Preferably, the envelope is made of an elastomeric material, such as latex or silicone rubber, and the inflatable chamber is substantially spherical or spheroidal, as shown in figure 1A.

The inflation tube 16 is sealed into the envelope 6. The inflation tube is preferably a rigid tube of metal or plastic having an outside diameter suitable for passing through a trocar. Alternatively, if a suitable manipulable introducer sleeve is used to place the inflatable retraction device in its collapsed state adjacent to the organ to be retracted, the inflation tube 16 can be made of a flexible material such as plastic or rubber.

The inflation tube 16 allows an inflation fluid to pass into and out of the inflatable chamber 11. The inflation fluid is preferably a gas, typically air, nitorgen or carbon dioxide, although a liquid, such as saline solution, or other suitable gases may be used. Typical inflation pressures are in the range 0.21 to 0.48 Pa (0.3 to 0.7 psi) the preferred pressure being 0.35 kPa (0.5 psi). Once the inflatable chamber is fully inflated, and the organ has been retracted, the inflation pressure can be reduced to about 0.21 kPa (0.3 psi) to maintain the organ in its retracted state. The proximal end of the inflation tube 16 is provided with a valve 21 which controls the flow of inflation fluid.

The inflatable retractor is shown in its collapsed state in figure 1B. The main envelope (not shown) is packed in a collapsed state, and is maintained in its packed state by the sleeve 26 and the detachable lacing 31. The sleeve 26 can alternatively be fitted with a tear strip (not shown) or detachable lacing can be used alone without a sleeve. As a further alternative, the packed main envelope can be accommodated within the inflation tube 16. The cord 36 releases the detachable lacing, but remains attached to the sleeve 26 so that cord can be used to withdraw the sleeve from the body.

### (b) Method of Using a Free Balloon Inflatable Retraction Device

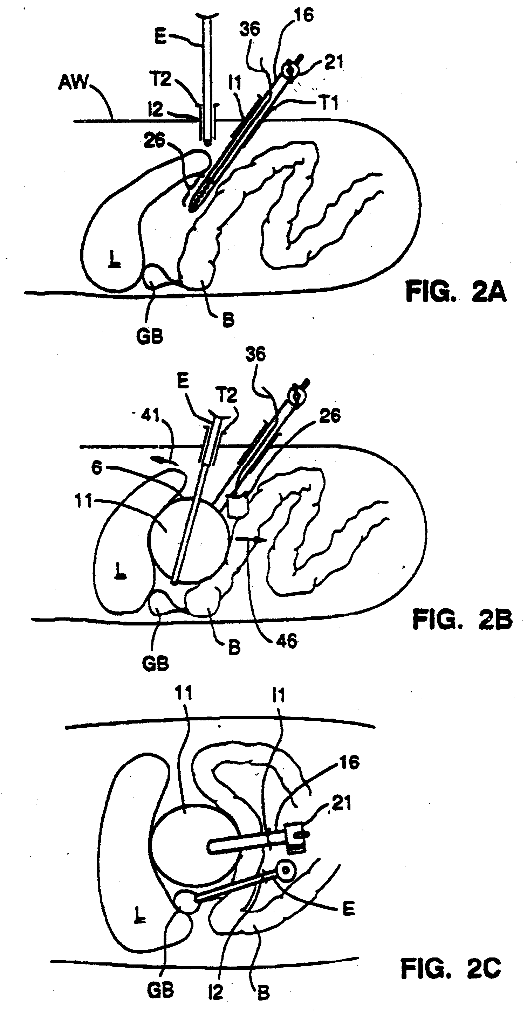

The method of using a free balloon inflatable retraction device according to the invention to separate the bowel and the liver to gain access to observe the gall bladder will now be described with reference to figures 2A and 2B.

The free balloon inflatable retraction device 1 is supplied with its envelope (not shown) picked in a collapsed state, and maintained in its collapsed state by the sleeve 26. Two small incisions I1 and I2, each about 10 - 20 mm long, are made in the abdominal wall AW, as shown in figure 2A. A trocar T1, suitable for receiving the free balloon inflatable retraction device 1 in its collapsed state, is driven through the incision I1 into the abdomen. An additional trocar T2, suitable for receiving the endoscope E, is drive through the incision I2 into the abdomen.

The endoscope E is inserted into the trocar T2 and positioned so that the intended deployment site of the free balloon inflatable retraction device 1 can be seen. The proximal end of the inflation tube 16 of the retractor is grasped and used to insert the distal end of the inflation tube and the packed envelope 29 of the inflatable chamber into the trocar T1. The inflation tube is advanced until the packaged envelope likes suitably positioned between the liver L and the bowel B, as shown in figure 2A.

The envelope 6 of the inflatable chamber is released from the sleeve 26 by pulling the cord 36 to detach the detachable lacing 31. A supply of a suitable inflation fluid (not shown) is connected to the inflation tube 16. The valve 21 is turned on to allow the inflation fluid to pass through the inflation tube into the inflatable chamber 11. The inflation fluid expands the inflatable chamber to its expanded state, as shown in figure 2B. The expanding inflatable chamber 11 lifts the liver L in the direction shown by the arrow 41, and retracts the bowel B in the direction indicated by the arrow 46.

The expanding inflatable chamber 11 of the free balloon inflatable retraction device 1 lifts the liver L by exerting a force against the bowel B, and retracts the bowel B by exerting a force against the liver L. Little, if any, of the retraction force is provided by the inflation tube 16.

The relative movement of the liver L and the bowel B opens up a passage through which the endoscope E can be advanced to observe the gall bladder GB, as shown in figure 2B. Figure 2C is a plan view showing the off-center placement of the incision I2 which allows the endoscope E to pass to the side of the inflatable chamber 11 to observe the gall bladder GB. Additional small incisions can be made to receive trocars through which other instruments can be inserted to treat the gall bladder.

## Claims

1. Apparatus for use inside the body for retracting a first tissue from a second tissue to gain access to a tissue adjacent the first tissue, said apparatus comprising inflatable chamber means (11) having a thin flexible main envelope (6) insertable into the body in a collapsed state through a small laparoscopic opening and inflating means (16) to inflate the envelope (6) to an expanded state while in place within the body to apply a force between the first tissue (e.g. L) and the second tissue (e.g. B) and retract the first tissue from the second tissue to provide access to the adjacent tissue (e.g. GB); characterized in that the apparatus further comprises detachable means (26) for maintaining the envelope (6) packed in the collapsed state while the envelope (6) is being inserted into the body through the laparoscopic incision.

2. Apparatus according to claim 1, characterized as additionally comprising means (36) for detaching the detachable means (26) prior to inflation of the envelope (6) to the expanded state.

3. The apparatus of claim 1, characterized in that the main envelope (6) includes a substantially non-elastic material.

4. The apparatus of claim 1, characterized in that the main envelope (6) includes an elastomeric material.

5. The apparatus of any of claims 1-4, characterized in that the inflating means (16) comprises a flexible tube sealed into the main envelope (6).

6. The apparatus of any of claims 1-4 characterized in that the inflating means (16) comprises a rigid tube means sealed into the main envelope (6).

7. The apparatus of claim 6, characterized in that the rigid tube means (16) is for providing little, if any, of the retraction force.

8. The apparatus of any of claims 1 through 7 characterized in that the inflatable chamber means (11) has a substantially spherical shape in the expanded state.

## Patentansprüche

1. Vorrichtung zur Verwendung innerhalb des Körpers zum Retrahieren eines ersten Gewebes von einem zweiten Gewebe, um Zugang zu einem Gewebe zu gewinnen, das dem ersten Gewebe benachbart ist, wobei die Vorrichtung eine aufpumpbare Kammereinrichtung (11), die eine dünne, flexible Haupthülle (6) hat und in den Körper in einem kollabierten Zustand durch eine kleine laparoskopische Öffnung einführbar ist, und eine Aufpumpeinrichtung (16) aufweist zum Aufpumpen der Hülle (6) in einen expandierten Zustand, während sie in dem Körper in Stellung ist, um eine Kraft zwischen dem ersten Gewebe (z.B. L) und dem zweiten Gewebe (z.B. B) auszuüben und das erste Gewebe von dem zweiten Gewebe zu retrahieren, um Zugang zu dem henachbarten Gewebe (z.B. GB) zu schaffen, dadurch gekennzeichnet, daß die Vorrichtung weiter eine lösbare Einrichtung (26) aufweist, um die Hülle (6) zusammengepackt in dem kollabierten Zustand zu halten, während die Hülle (6) durch den laparoskopischen Schnitt in den Körper eingeführt wird.

2. Vorrichtung nach Anspruch 1, dadurch gekennzeichnet, daß sie zusätzlich eine Einrichtung (36) aufweist zum Lösen der lösbaren Einrichtung (26) vor dem Aufpumpen der Hülle (6) in den expandierten Zustand.

3. Vorrichtung nach Anspruch 1, dadurch gekennzeichnet, daß die Haupthülle (6) ein im wesentlichen unelastisches Material aufweist.

4. Vorrichtung nach Anspruch 1, dadurch gekennzeichnet, daß die Haupthülle (6) ein elastomeres Material aufweist.

5. Vorrichtung nach einem der Ansprüche 1-4, dadurch gekennzeichnet, daß die Aufpumpeinrichtung (16) ein flexibles Rohr aufweist, das in die Haupthülle (6) eingeschweißt ist.

6. Vorrichtung nach einem der Ansprüche 1-4, dadurch gekennzeichnet, daß die Aufpumpeinrichtung (16) eine starre Rohreinrichtung aufweist, die in die Haupthülle (6) eingeschweißt ist.

7. Vorrichtung nach Anspruch 6, dadurch gekennzeichnet, daß die starre Rohreinrichtung (16) zum Erzeugen eines geringen Teils, wenn überhaupt, der Retraktionskraft vorgesehen ist.

8. Vorrichtung nach einem der Ansprüche 1 bis 7, dadurch gekennzeichnet, daß die aufpumpbare Kammereinrichtung (11) in dem expandierten Zustand eine im wesentlichen sphärische Form hat.

## Revendications

1. Appareil à utiliser à l'intérieur du corps pour séparer un premier tissu d'un second tissu pour avoir un accès à un tissu adjacent au premier tissu, cet appareil comprenant une chambre gonflable (11) comportant une mince enveloppe (6) principale flexible pouvant être insérée dans le corps dans un état plié au travers d'une petite ouverture laparoscopique et un dispositif de gonflage (16) pour gonfler l'enveloppe (6) vers son état gonflé alors qu'elle est en place à l'intérieur du corps pour appliquer une force entre le premier tissu (p.ex. L) et le second tissu (p.ex. B) et séparer le premier tissu du second tissu pour créer un accès vers le tissu adjacent (p.ex. GB), caractérisé en ce que l'appareil comprend en outre un dispositif détachable (26) pour maintenir l'enveloppe (6) compactée dans l'état replié alors que l'enveloppe (6) est insérée dans le corps au travers de l'incision laparoscopique.

2. Appareil selon la revendication 1 caractérisé en ce qu'il comprend un dispositif supplémentaire (36) pour détacher le dispositif détachable (26) avant le gonflage de l'enveloppe (6) vers son état gonflé.

3. Appareil selon la revendication 1 caractérisé en ce que l'enveloppe principale (6) comprend une matière sensiblement non élastique.

4. Appareil selon la revendication 1 caractérisé en ce que l'enveloppe principale (6) comprend une matière élastomère.

5. Appareil selon l'une quelconque des revendications 1-4, caractérisé en ce que le dispositif de gonflage (16) comprend un tube flexible scellé dans l'enveloppe principale (6).

6. Appareil selon l'une quelconque des revendications 1-4, caractérisé en ce que le dispositif de gonflage (16) comprend un tube rigide scellé dans l'enveloppe principale (6).

7. Appareil selon la revendication 6 caractérisé en ce que le tube rigide (16) fournit peu, s'il y en a, de force de rétraction.

8. Appareil selon l'une quelconque des revendications 1-7 caractérisé en ce que la chambre gonflable (11) a une forme sensiblement sphérique dans son état gonflé.
